# EUROPEAN PATENT SPECIFICATION

(11) **EP 3 068 281 B1**
(45) Date of publication and mention of the grant of the patent: **17.01.2018**
(21) Application number: 14809285.1
(22) Date of filing: 10.11.2014
(51) Int. Cl.: A61B 1/31, A61B 5/06, G06T 7/00

(54) **AN APPARATUS FOR ENDOSCOPY**
VORRICHTUNG FÜR ENDOSKOPIE
APPAREIL POUR ENDOSCOPIE

(30) Priority: 12.11.2013 DK 201300642
(43) Date of publication of application: 21.09.2016
(73) Proprietor: Simonsen & Weel A/S, 2665 Vallensbæk Strand (DK)
(72) Inventor: SVENDSEN, Lars B., 2840 Holte (DK); SVENDSEN, Morten B., 2100 København Ø (DK); KONGE, Lars, 2830 Virum (DK)
(74) Representative: Rasmussen, Martin Hoffgaard
(86) International application number: PCT/DK2014/000052
(87) International publication number: WO 2015/070866

(56) References cited:
- EP-A1- 2 215 960
- WO-A1-2009/128055
- WO-A1-2013/154764
- WO-A2-2012/082464
- US-A1- 2006 293 558

## Description

### Field of the invention

The present invention relates in a first aspect to an apparatus for monitoring the quality of an endoscopy. In a second aspect, the present invention relates to the use of an apparatus according to the first aspect for training a medical staff person in performing an endoscopy. In a third aspect the present invention relates to a method of training a medical staff person in performing an endoscopy.

### Background of the invention

Complications in the large intestine (colon) seem to be an increasingly occurring pathological condition among people in the western world. These complications may range from non-fatal conditions, such as colon irritabile, colitis ulcerosa, diverticulitis, polyps to more severe and potentially fatal conditions such as cancer in the large intestine (cancer coli).

In many of these conditions a medical diagnosis will include a visual inspection of the interior of the large intestine. To this end a doctor will use an endoscope which comprises an elongate flexible tubing having a proximate end and a distal end. The distal end is adapted to be inserted into the large intestine of the patient via the rectum. At the distal end of the endoscope is provided image capturing means such as a camera or a lens capturing and transmitting images of the interior of the intestine to a monitor. At the proximate end of the endoscope are provided shape controlling means for allowing a doctor to adjust the spatial shape of said tubing in such a way that the shape of the tubing essentially follows the shape of the interior of the patient's intestine.

By inserting the endoscope into the patient's rectum and by adjusting the shape of the endoscope using the shape controlling means at the proximate end of the endoscope a doctor will be able to lead the distal end of the endoscope through the large intestine and along the way the doctor may inspect the images displayed on the monitor and representing the appearance of the interior of the large intestine at the point of the distal end of the endoscope. Such visual inspections are of great help in allowing a doctor to arrive at a correct diagnosis associated with a specific patient.

The human large intestine is having a rather complicated shape (extending in the direction from the end of the rectum to the small intestine) first comprising a small downward bend followed by an upward extension in the left hand side of the individual to a position to the left of the stomach. At this position the large intestine makes a sharp bend and extends just below the stomach towards the right hand side of the individual. At a position located right relative to the stomach the large intestine takes yet another sharp bend, this time in a downward direction where it finally meets the end of the small intestine.

If a doctor is to visually inspect the appearance of the large intestine of an individual by using an endoscope he will have to insert the endoscope into the individual via that individual's rectum and he will have to attempt to adjust the endoscope using the shape controlling means at the proximate end of the endoscope in such a way that the shape of the endoscope essentially follows the topography of the interior of the individual's intestine.

The wall of the human intestine is highly sensitive towards mechanical impacts such as those imposed by medical or diagnostic instruments inserted into the interior of the large intestine during medical surgery or diagnosis. An individual will accordingly almost always experience a colonoscopy as ranging from somewhat painful and unpleasant to extremely uncomfortable.

Hence, a successful endoscopic inspection of the large intestine is difficult and requires great skills and practice of the endoscopist performing it.

To this must be added that it is not uncommon that an endoscopy of the large intestine accidentally will lead to a rupture of the large intestine. Approximately one in every two thousands of all endoscopies leads to a perforation of the intestine. Up to one third of these perforations have fatal outcome, leading to the death of the individual being examined.

Accordingly, the above described traditional way of performing an endoscopy by inserting an endoscope into the large intestine via rectum and solely being based on the doctor's skills in feeling and sensing how the endoscope is to be controlled so as to adapt the shape of the endoscope to the topography of the interior wall of the large intestine was not an optimum solution for the individual being subject to the inspection.

In recent years an improved version of a traditional endoscopic apparatus has been developed and marketed by Olympus as the UPD system. The Olympus system is generically denoted as magnetic endoscopic imaging. The magnetic endoscopic imaging system comprises an elongate flexible endoscopic arm comprising image capturing means such as a camera capturing images of the interior of the intestine, as described above. Further, the elongate flexible endoscopic arm of the magnetic endoscopic imaging system comprises a number of magnets arranged with a predefined mutual distances along the length of the arm, as described above in respect of a traditional endoscopic arm. Finally, the proximate end of the endoscope arm is provided with shape controlling means for allowing a doctor to adjust the spatial shape of the elongate flexible endoscopic arm as described above in respect of a traditional endoscopic arm.

The magnetic endoscopic imaging system additionally comprises one or more sensor coils configured to be arranged outside a patient and being adapted to sense the positions of one or more of the magnets arranged on the elongate flexible endoscopic arm.

A control unit is receiving information associated with the electric signals received by the sensing coil(s) and the control unit transforms this information to a 3D representation of the positions of individual magnets of the endoscopic arm and this 3D representation is displayed on a monitor. Accordingly, the displayed 3D representation of the coils will provide real time information relating to the actual shape of the endoscopic arm in the interior of an individual's intestine. Also displayed on a monitor are the images captured by the image capturing device arranged on the distal end of the endoscope.

In Denmark the National Health and Medicines Authority (Sundhedsstyrelsen) on the basis of governmental legislation adopted in 2011 has launched an initiative for strengthening early diagnosis of colorectal cancer. The practical implementation of this initiative will commence in 2014.

This initiative will include inviting every individual of age of 50 - 74 years to a screening for colorectal cancer. The screening will imply a faecal analysis for blood and if positive a preliminary interview with a doctor combined with providing a blood sample from that individual in order to assess whether the specific individual is i) exhibiting changed patterns, such as changed regularity patterns in bowel movements, ii) exhibiting blood in stool, iii) exhibiting loss of weight and iv) having a blood deficiency.

All cases exhibiting a positive faecal blood test will be subjected to a further examination in the form of an endoscopic of the large intestine, thus allowing the medical staff to obtain a visual inspection of the interior of the large intestine of that individual.

It is believed that, based on the basis of the new diagnosis initiative, the annual increase in number of performed colonoscopies nationwide in Denmark will amount to 100 % within the first two years of practical implementation of the new diagnosis initiative.

It is believed that other jurisdictions will soon be initiating similar screening programs for discovering pathological conditions in the colon with the view to cure any diseases at an earlier stage.

Although the currently existing magnetic endoscopic imaging system have proven to represent great improvement relative to the traditional way of performing a endoscopy, this system never the less represents a number of deficiencies.

One of these deficiencies is that although that the currently existing magnetic endoscopic imaging system provides a real time indication of the actual spatial shape of endoscopic arm displayed on a monitor, there are no instructions provided by that system to the medical staff relating to an ideal and thus safe and least unpleasant mode of insertion of the endoscope such as for example relating to inter alia speed of insertion at various points in the large intestine, residence time of the endoscope at various points in the large intestine.

It is clear that the statistical fact that one in every 2000 individual being subjected to a colonoscopy tragically will experience a perforation of the intestine with a high risk of subsequently death of that individual, calls for an optimized training of the medical staff performing the colonoscopy in order to improve the skills of that medical staff and thereby improving the overall quality of the colonoscopy offered by that medical staff. Furthermore, it should be noted that a not insignificant amount of the individuals screened turns out at the time of screening to be healthy persons without symptoms.

Another deficiency of the currently existing magnetic endoscopic imaging system is that upon terminating an endoscopic insertion, the medical staff will be provided with no objective assessment as to the quality of that endoscopy. Hence, the existing magnetic endoscopic imaging system does not provide an objective assessment of the skills of the specific medical staff person who had conducted the endoscopy.

Accordingly, improvements in the technology of magnetic endoscopic imaging apparatuses for performing colonoscopies are much needed.

It is the object of the present invention to overcome such deficiencies.

WO-A-2009/128 055 discloses an apparatus for monitoring the quality of an endoscopy performed on an individual, said apparatus comprising an endoscopic arm, a sensor system adapted to be arranged outside the body of an individual and a quality assessment system for evaluating the quality of a specific endoscopy procedure performed on an individual.

### Brief description of the invention

The above mentioned disadvantages are alleviated by the present invention in its first, second and third aspect, respectively.

In its first aspect, the present invention relates to an apparatus for monitoring the quality of an endoscopy, in particular a colonoscopy, performed on an individual, according to claim 1. In its second aspect, the present invention relates to a use of the apparatus according to the first aspect of the present invention for training a medical staff person in performing an endoscopy, especially a colonoscopy.

In its third aspect, the present invention relates to a method for training a medical staff person in performing an endoscopy, especially colonoscopy, according to claim 15. The present invention in its first, second and third aspects, respectively provides an apparatus, a use and a method for improvement in the quality of an endoscopic insertion of an endoscope in an individual. Accordingly, the various aspects of the present invention may provide instructions to the medical staff relating to an ideal and thus safe and least unpleasant mode of insertion of the endoscope such as for example relating to inter alia speed of insertion at various points in the large intestine, residence time of the endoscope at various points in the large intestine.

Furthermore, the present invention in its first, second and third aspects, respectively provides for an optimized training of the medical staff performing the colonoscopy in order to improve the skills of that medical staff and thereby improving the overall quality of the colonoscopy offered by that medical staff.

Finally, present invention in its first, second and third aspects, respectively provides for an objective assessment as to the quality of an endoscopy.

### Drawings

Fig. 1 illustrates schematically the gastro-intestinal tract of a human individual.
Fig. 2 illustrates schematically one embodiment of an apparatus according to the present invention.
Fig. 3 shows details of an elongate flexible endoscopic arm for use with the apparatus according to the present invention.
Fig. 4 illustrates one embodiment of an algorithm for calculating a score representing the quality of an endoscopic insertion.
Fig. 5a is a diagram showing the various positions of a tip of an endoscope during an endoscopic insertion in a large intestine, performed by an unskilled medical staff person.
Fig. 5b depicts histograms representing the scores of the insertion of the endoscope as represented by fig. 5a.
Fig. 6a is a diagram showing the various positions of a tip of an endoscope during an endoscopic insertion in a large intestine, performed by a skilled medical staff person.
Fig. 6b depicts histograms representing the scores of the insertion of the endoscope as represented by fig. 6a.
Fig. 7 depicts two examples (one of an experienced and one of a unexperienced) of a colonoscopy progression map obtained with the apparatus according to the first aspect of the present invention and used in the example.
Fig. 8 shows distributions of colonoscopy progression scores in an easy model intestine (Fig. 8a) and in a difficult model intestine (Fig. 8b) as obtained in the example.

### Detailed description of the invention

### The first aspect of the present invention

In a first aspect, the present invention relates to an apparatus for monitoring the quality of an endoscopy, in particular a colonoscopy, performed on an individual, said apparatus comprises:
- an elongate flexible endoscopic arm adapted to be inserted into the large intestine of an individual; and
- a sensor system adapted to be arranged outside the body of an individual; and
- a quality assessment system for evaluating the quality of a specific endoscopy procedure performed on an individual;
wherein said endoscopic arm is being adapted to be inserted into the large intestine of an individual via that individual's rectum, said endoscopic arm having a proximate end and a distal end;
wherein said endoscopic arm at a position near the proximal end comprises shape controlling input means for controlling the spatial shape of said arm; and
wherein said endoscopic arm along its extension comprises means for adjusting the spatial shape of said arm in response to an activation of said shape controlling input means;
wherein said endoscopic arm along its extension comprises one or more position indicating means, each position indicating means being adapted to provide information as to the position of said specific position indicating means; and
wherein said endoscopic arm near its distal end comprises one or more image capturing means for capturing images of the interior of an intestine;
wherein said sensor system being adapted for sensing one or more of said position indicating means of said endoscopic arm; said sensor system being adapted to generate an electrical signal as a representation of the position of one or more of said position indicating means, when said one or more position indicating means are within a detection range of said sensor system;
wherein said sensor system comprises a control unit comprising a microprocessor which is adapted to process the electrical signal originating from said sensor system, said control unit being adapted to translate this electrical signal into a 3D representation of the position of one or more of said position indicating means in the form of a monitor signal;
wherein said apparatus comprises a monitor, said monitor being adapted to display graphically said 3D representation of the position of one or more of said position indicating means;
wherein said apparatus comprises a monitor, said monitor being adapted to display graphically the imaged captured by the one or more image capturing means of said endoscopic arm;
wherein said quality assessment system comprises means for determining, at predefined time intervals, the position in the three dimensional space, of one or more selected points; said one or more selected points being points fixed on the elongate flexible endoscopic arm;
wherein said quality assessment system comprises means for determining, in respect of one or more of said selected points, one or more parameters relating to the progression of one or more of said selected points; said parameters being selected from the group comprising: speed of progression of a specific selected point, direction of the progression of a specific selected point, time span of non-movement of a specific selected point;
wherein said quality assessment system comprises a data storage for storing data associated with parameters of one or more examples of an ideal progression of an endoscopic insertion;
wherein said quality assessment system comprises means for calculating one or more scores by comparing one or more parameters of an actually performed endoscopy insertion with the same one or more parameters associated with one or more of said ideal progressions of an endoscopic insertion, wherein said one or more scores representing the degree of deviation from one or more selected examples of an ideal progression of an endoscopic insertion;
wherein said quality assessment system comprises communication means which is adapted to communicate said one or more scores to the user.

In one embodiment of the first aspect of the present invention, one of said one or more selected points being a point located at the distal end of the elongate flexible endoscopic arm.

Such an embodiment will allow a quality assessment of the progression of the flexible endoscopic arm, at least as represented by the progression of a distal point of said arm, such as the tip of said arm.

In one embodiment of the first aspect of the present invention said one or more position indicating means of said endoscopic arm comprises permanent magnets or electromagnets.

Such types of the position indicating means of said endoscopic arm provides for easy and accurate position determination by means of e.g. detector coils of the sensor system of the apparatus.

In one embodiment of the first aspect of the present invention the number of position indicating means arranged along the extension of the endoscopic arm is 1 - 50, such as 2 - 48, such as 4 - 46, e.g. 6 - 44, for example 8 - 42, such as 10 - 40, such as 12 - 38, such as 14 - 36, e.g. 16 - 34, for example 18 - 32, such as 20 - 30, such as 22 - 28, for example 24 - 26.

In general, the higher the number of position indicating means present along the extension of the endoscopic arm, the more details of a 3D image of the arm may be reproduces on the monitor intended to graphically display said 3D representation of positions of the position indicating means. The above numbers of position indication means have proven suitable in this respect.

In one embodiment of the first aspect of the present invention one or more image capturing means of the endoscopic arm for capturing images of the interior of an intestine being in the form of a video camera or a lens comprising means for transmitting images to said control unit.

In one embodiment of the first aspect of the present invention said elongate flexible endoscopic arm comprises one or more illuminating means for illuminating the interior of an intestine, said one or more illuminating means preferably being arranged at the distal part of said elongate flexible endoscopic arm.

Illumination means allow the image capturing mean(s) arranged on the distal part of the arm to capture higher quality of images.

In one embodiment of the first aspect of the present invention said monitor being adapted to display graphically said 3D representation of the position of one or more of said position indicating means is the same monitor or is a different monitor as that monitor being adapted to display graphically the images captured by the one or more image capturing means of said endoscopic arm.

Such an embodiment ensures reduction of the numbers of monitors to be used, and hence saves space.

In one embodiment of the first aspect of the present invention said data associated with parameters of one or more examples of an ideal progression of an endoscopic insertion are based on a single human individual; or said data associated with parameters of one or more examples of an ideal progression of an endoscopic insertion are based on an average of a number of different human individuals.

If the data associated with parameters of one or more examples of an ideal progression of an endoscopic insertion are based on a single human individual it will be possible to adapt the actual insertion of the endoscopic arm to that that specific human individual, thus being able to take care of that individual's specific intestine topography. However, generally, the data associated with parameters of one or more examples of an ideal progression of an endoscopic insertion are based on an average of a number of different human individuals.

In one embodiment of the first aspect of the present invention said communication means of said quality assessment system, which is adapted to communicate said one or more scores to the user, is configured to communicate said one or more scores graphically, such as by displaying colour(s) and/or by displaying numbers, preferably on a monitor.

In one embodiment of the first aspect of the present invention said apparatus is configured so as to calculate the position of one or more of said selected points in the three dimensional space directly from the information provided by the said electric signal originating from said sensor system; or indirectly from said monitor signal provided by said control unit.

These embodiments allow, in the calculation of one or more of said selected points in the three dimensional space, the most direct transformation of signals detected by said sensor system on the one hand, or an indirect calculation based on monitor provided by said control unit, on the other hand.

In one embodiment of the first aspect of the present invention said data associated with parameters of one or more examples of an ideal progression of an endoscopic insertion and being stored on said data storage is defined as: shortest possible time to the coecum, with no clinical obvious complain from the individual or with complain from the individual below a predefined level.

In this embodiment the ideal progression of an endoscopic insertion represents an insertion having the least uncomfortable impact on the individual on whom the insertion is made.

In one embodiment of the first aspect of the present invention said apparatus comprises a data storage 52 for recording, as a function of time, the position of one or more of said position indicating means.

Such data storage allows for performing an analysis relating to the quality of the insertion of the endoscope in the large intestine.

In one embodiment of the first aspect of the present invention said apparatus comprises input means for inputting the identity of the specific medical staff person, performing a specific endoscopy; and wherein said apparatus optionally furthermore comprises means for correlating and displaying, in respect of said specific medical staff person, an average score obtained by said specific medical staff, thus allowing obtaining a accumulated quality assessment of one or more medical staff persons.

This embodiment allows keeping a statistical record of the quality of endoscopic procedures performed by a range of different persons belonging to the medical staff.

In one embodiment of the first aspect of the present invention the apparatus is an apparatus for training a medical staff person in performing an endoscopy as an alternative of being an apparatus for monitoring the quality of an endoscopy.

Generally, the first aspect of the present invention may alternatively relate to the apparatus as defined in claim 1 with the alternative design that the feature that "*said quality assessment system comprises means for calculating one or more scores by comparing one or more parameters of an actually performed endoscopy insertion with the same one or more parameters associated with one or more of said ideal progressions of an endoscopic insertion, wherein said one or more scores representing the degree of deviation from one or more selected examples of an ideal progression of an endoscopic insertion*" is an optional feature.

In this alternative first aspect, the quality assessment system comprises means for calculating one or more scores based on the deviation of one or more parameters of an actually performed endoscopy insertion with the same one or more parameters associated with a predetermined mode of progression of an endoscopic insertion.

In this way a score may be calculated based on comparison with parameters of any kind of a predetermined endoscopic insertion.

### The second aspect of the present invention

In a second aspect, the present invention relates to a use of the apparatus according to the first aspect of the present invention for training a medical staff person in performing an endoscopy, especially a colonoscopy.

In one embodiment of the second aspect of the present invention said use is for training a medical staff person in performing an endoscopy on an individual, said individual being a model of a human being or part of a model of a human being or model of a part of a human being.

For untrained medical staff it would advantageous for practice purposes to perform the insertion of an endoscope on a non-live individual, such as a model of a human being or part of a model of a human being, or model of a part of a human being. However, the use may also be performed on a live human being.

Accordingly, in the present description and in the appended claims, an individual may be construed to mean an non-live and/or artificial model of a human being or part of a model of a human being or a model of a part of a human being.

Such use is particularly relevant for solely endoscopic insertion practicing purposes.

### The third aspect of the present invention

In a third aspect, the present invention relates to a method for training a medical staff person in performing an endoscopy, especially colonoscopy, said method comprising:
i) providing an apparatus according to the first aspect of the present invention;
ii) inserting the distal end of the elongate flexible endoscopic arm into the rectum of an individual;
iii) progressively inserting the elongate flexible endoscopic arm into that individual's large intestine while following the internal topography of the large intestine by means of adjustment of the shape controlling input means of the endoscopic arm, and with reference to the 3D representation of the position of one or more of said position indicating means on said monitor;
iv) during step iii) repeatedly receiving information from said quality assessment system relating to the degree of deviation from progression of an endoscopic insertion, said information being provided by said score/scores;
v) using the score/scores obtained in step iv) as a feed-back for correcting the progression of the progression of the insertion of the endoscopic arm, and subsequently, if necessary, correcting the progression of the insertion of the endoscopic arm;
vi) retracting said endoscopic arm from the individual's body;
   with the provisio that said individual being a model of a human being or part of a model of a human being or model of a part of a human being. This embodiment allows use of the information gained during the endoscopy to form basis of a diagnosis of the individual being subject to the endoscopy.

In one embodiment of the third aspect of the present invention individual being a model of a human being or part of a model of a human being or model of a part of a human being.

For untrained medical staff it would advantageous for practice purposes to perform the insertion of an endoscope on a non-live individual, such as a model of a human being or part of a model of a human being or model of a part of a human being. However, the method may also be performed on a live human being.

In one embodiment of the third aspect of the present invention said method does not involve a diagnosis or a surgical step.

Such method is particularly relevant for solely endoscopic insertion practicing purposes.

In the present description the term "endoscopic insertion" shall be interpreted to mean either an insertion of an endoscope into an individual's intestine, or an extraction of an endoscope from an individual's intestine, or a combination of an insertion followed by an extraction of an endoscope from an individual's intestine, or parts of such insertions and exertions or combinations thereof.

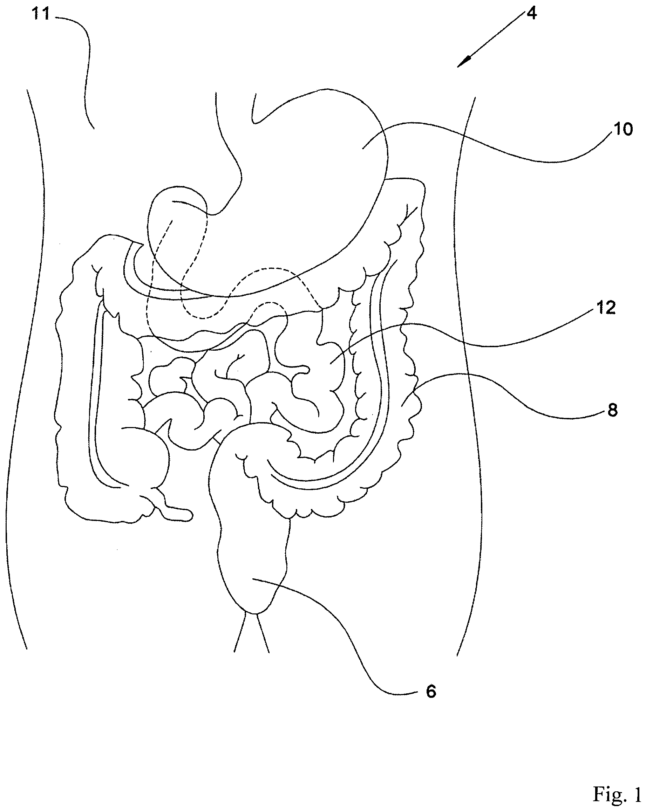

Referring now in details to the drawings for the purpose of illustrating preferred embodiments of the present invention, a gastro-intestinal tract of a human individual is schematically outlined in fig. 1.

Fig. 1 shows the abdominal part 4 of a human individual 11. The gastro-intestinal tract comprises (in the direction of digestive movements) the stomach 10, followed by the small intestine 12, followed by the large intestine 8 and ending in rectum 6. Fig. 1 shows clearly the curved progression of the large intestine comprising various bends, kinks and curved topography. From fig. 1 it will be clear that progressively inserting an endoscope into the large intestine requires a decent amount of skills.

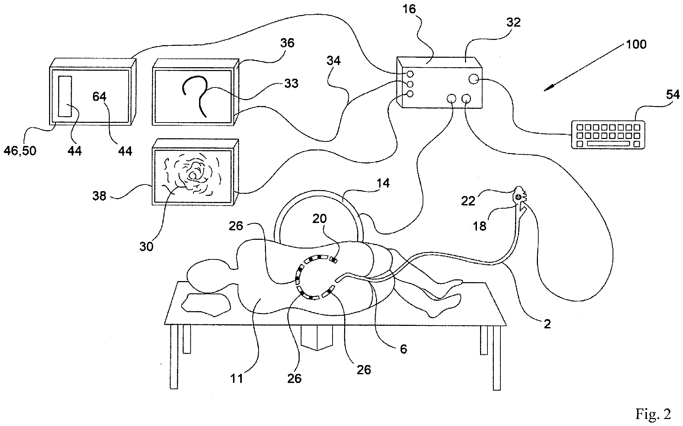

Fig. 2 shows schematically one embodiment of an apparatus 100 according to the first aspect of the present invention. In fig. 2 is shown an individual 11 being prepared for colonoscopy by inserting a distal part 20 of an endoscope 2 into the individuals large intestine via rectum 6. The part of the endoscope 2 being inserted into the large intestine comprises along its extension a number of position indicating means 26 adapted to provide information as to where in the three dimensional space each of these position indicating means 26 are located.

Further, the distal end of the elongate flexible arm comprises image capturing means 20 for capturing images if the interior of an intestine, shown on monitor 38.

In the opposite end 18 of the endoscope are provided shape controlling input means 22. These shape controlling input means allow a user to control the spatial shape of the elongate wire. Further, the endoscope comprises means 24 (not shown) for adjusting the spatial shape of endoscopic arm. These means 24 adapt the shape of the elongate flexible arm as a response to the input provided via the shape controlling input means 22 or by torqueing the endoscope.

Outside the individual 11 are arranged a sensor system 14 here illustrated as comprising a single detector coil 14. However, a number of detector coils may be present in the sensor system 14.

The sensor system 14 senses the presence and location of each of the position indicating means 26 on the elongate, flexible endoscopic arm 2

A control unit 32 receives signals from the sensing system 14. These signals are electronically processed so as to be translated into a 3D representation of the position of one or more of said position indicating means in the form of a monitor signal 34. This monitor signal is being transmitted to monitor 36 showing this 3D representation of the position of position indicating means 26.

The control unit 32 also controls the signals captured by the image capturing means 26 and process these so as to transform them into a monitor signal which is being send to monitor 38 which in turn depicts the image 30 of the intestine as seen from within said intestine.

Finally, the control unit 32 has embedded in it a data storage 42 (not seen in fig 2) for storing data associated with parameters of one or more examples of an ideal progression of an endoscopic insertion.

The control unit furthermore comprises a quality assessment system 16 which comprises means for determining, at predefined time intervals, the position in the three dimensional space, of one or more selected points 40, wherein said one or more selected points 40 being points fixed on the elongate flexible endoscopic arm.

Thereby it will be possible for the quality assessment system 16 to determine, in respect of one or more of said selected points 40, one or more parameters relating to the progression of one or more of said selected points; said parameters being selected from the group comprising: speed of progression of a specific selected point, direction of the progression of a specific selected point, time span of non-movement of a specific selected point.

The quality assessment system 16 also comprises means for calculating one or more scores 44 by comparing one or more parameters of an actually performed endoscopy insertion with the same one or more parameters associated with one or more of said ideal progressions of an endoscopic insertion, wherein said one or more scores representing the degree of deviation from one or more selected examples of an ideal progression of an endoscopic insertion. In fig. 2 such score or scores are displayed on the communication means 46 in the form of a monitor 50, as vertical bar and the numeral, respectively 44.

Also seen in fig. 2 is the input means 54 allowing a user to input his identity for the purpose of obtaining an accumulated score representing the skills of the user in performing endoscopy.

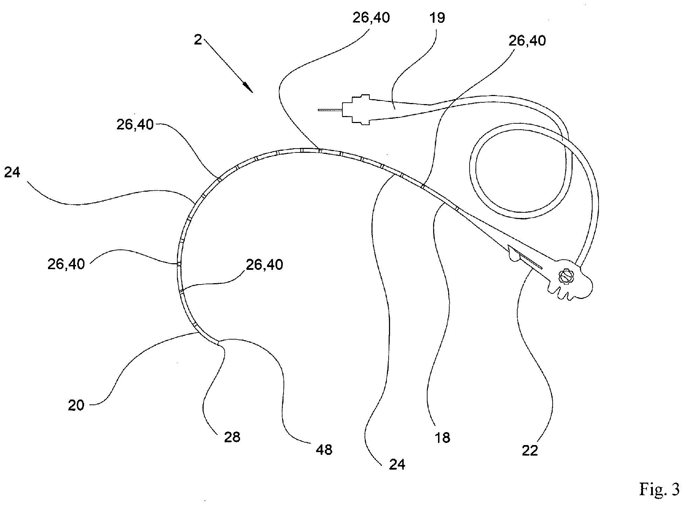

Fig. 3 shows details of the endoscopic arm 2 of the apparatus 100 according to the first aspect of the present invention. The arm 2 comprises an elongate flexible wire or the like having a proximal end 18 and a distal end 20. The distal end is configures to be inserted into an individual's intestine. The distal end comprises image capturing means 28 and may also comprise illuminating means 48 for illuminating the interior of the intestine.

In the other end of the elongate, flexible wire, the proximal end 18, are provided a handle comprising shape controlling input means 22. These shape controlling input means allow a user to control the spatial shape of the elongate wire so as to adapt that shape to the intestine in which the endoscope is inserted. From the handle also extends a connector 19 for connecting to control unit 32. Along the extension of the elongate flexible wire are provided a number of position indicating means 26. These position indicating means may be permanent magnets or electro magnets providing a magnetic field and thus providing information as to where in the three dimensional space each of these position indicating means 26 are located.

In fig. 3 are also shown a number of selected points 40 on elongate flexible endoscopic arm. The positions of these selected points 40 may be identical to the positions of the position indicating means 26 or they may be different. The selected points 40 being points in respect of which e.g. the position, the speed, the direction of movement, and the time of non-movement may be detected and recorded for the purpose of identifying the characteristics of progression of the elongate flexible endoscopic arm into an individual's intestine.

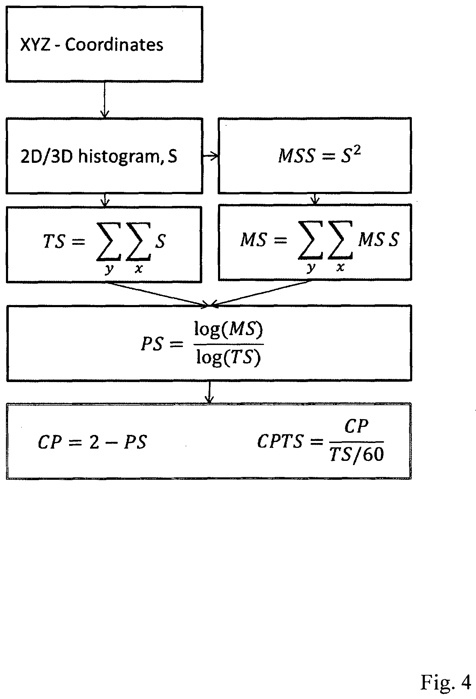

Fig. 4 illustrates one example of an algorithm for evaluating the quality of a progression of an endoscopic insertion. Fig 4 is a flow diagrammatic representation of how the data relating to the progression of an insertion of the tip of an endoscopic arm may be processed and converted into a score representing the quality of that progression.

In the acquisition of the data representing the position of the tip of an endoscopic arm, the position of a position indicating means positioned at the tip of said arm is determined by the sensor system and the control unit at a sampling rate of e.g. 12 determinations per second. This sampled data is denoted as the "XYZ - Coordinates" in fig. 4.

This data is subsequently quantified into a histogram representing, in respect of each bin of s given and predefined size of the quantified space, the number of samples in which the tip is located within said specific bin. This quantification is in fig. 4 represented by the box titled "2D/3D histogram, S".

In order to provide for a reduction in the final score, in case there is too slow progression in a given bin, the quantification S is squared to S². This is shown in the box in fig. 4 titled MSS= S². The term MMS denotes "Matrix single squared".

In respect of the histograms titled S and MSS, all bins are summarized; first in respect of the y axis and subsequently in respect of the x axis. The summarizing is performed in respect of S and MSS, respectively. This will result in a time score TS and a matrix score MS as denoted by the two boxes in the middle row in fig. 4.

Subsequently, the time score and the matrix score is used as a basis for calculating a progression score PS according to the equation: PS = log(MS)/log(TS).

The progression score will be a number in the range of [1;2]. In order to arrive at a score in the range [0;1], the progression score PS is converted to the coloscopy progression score, CP, defined as CP = 2 - PS.

Additionally, in order to correlate the progression in relation to time, a coloscopy progression time score, CPTS is calculated. The value of CPTS is calculated as: CPTS = CP/ (TS/60). The CPTS represents the quality of a specific endoscopy performed compared to the time spent in performing said endoscopy.

It should be noted that in the acquisition of the data representing the position of a position indicating means arranged on an endoscopic arm, by the sensor system and the control unit, the data may for calculating the position of one or more of said selected points 40 in the three dimensional space directly from the information provided by the said electric signal originating from said sensor system 14; or indirectly from said monitor signal 34 provided by said control unit 32. Reference is made to the literature relating to image analysis, such as in Rangaraj M. Rangayyan, Biomedical Image Analysis, CRC Press, 2005. ISBN: 0-8493-9695-6, Chapters 2, 5 and 6.

The algorithm shown in fig. 4 is merely one of a number of possible algorithms useful for analyzing insertion data and for converting these date to an expression representing the quality of a performed endoscopy. A skilled person in the art will be able to deduce further and/or alternative algorithms.

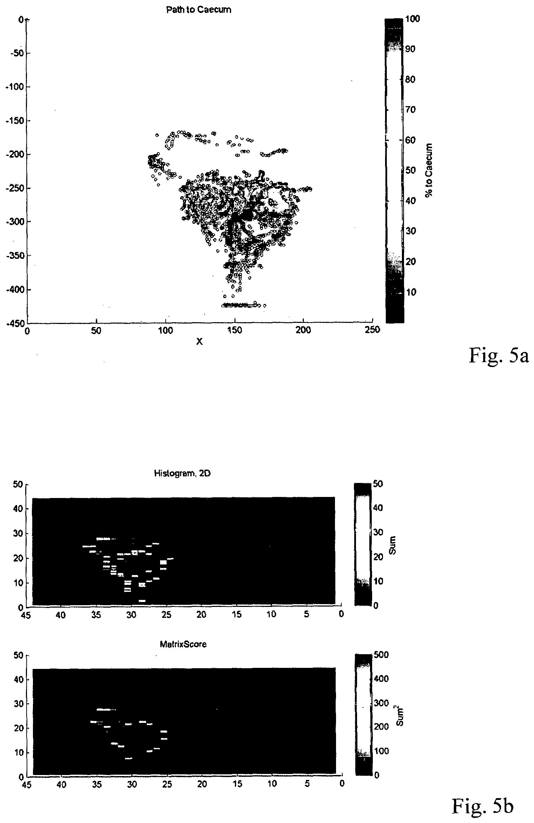

Fig. 5a illustrates a quantified representation of the various positions of the tip of an endoscopic tip during insertion by a not so skilled medical staff person. It can be seen that the path is not very well defined as if the endoscopist is feeling his way through the large intestine. The X and Y axis represent spatial parameters and the colors represent "percent of procedure" where blue indicates beginning and red indicates end of insertion.

The algorithm depicted in fig. 4 was applied in order to make a quality assessment of the colonoscopy represented by fig. 5a.

The result of following this algorithm is depicted in fig. 5b, which shows a histogram and the associated matrix score. The CP value of the colonoscopy represented by fig. 5a was approximately 49.

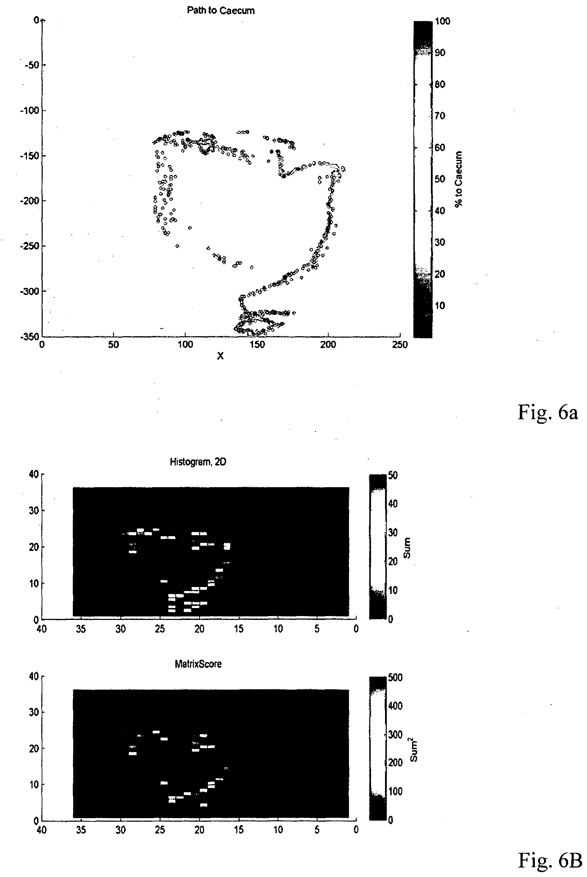

Fig. 6a illustrates a quantified representation of the various positions of the tip of an endoscopic tip during insertion, this time by a skilled medical staff person. Compared to fig. 5a it can be seen that the path is much more well defined as if the endoscopist know his way way through the large intestine. The X and Y axis represent spatial parameters and the colors represent "percent of procedure" where blue indicates beginning and red indicates end of insertion.

The algorithm depicted in fig. 4 was applied in order to make a quality assessment of the colonoscopy represented by fig. 6a.

The result of following this algorithm is depicted in fig. 6b, which shows a histogram and the associated matrix score. The CP value of the colonoscopy represented by fig. 6a was approximately 25.

It should be noted that a variety of other kinds of algorithms for calculating a score of an endoscopic insertion is possible. A person skilled in the art will be in a position to come up with such alternative algorithms.

### Example

This example demonstrates the evaluation of a colonoscopy performance by using an apparatus according to the present invention.

Ten experienced endoscopy consultants with a minimum experience of 350 colonoscopies (median 2000, range 350-4000) participated in the example. Additionally, eleven trainees participating in a simulator-based colonoscopy training program were recruited for comparison.

Trainees who had previously received formal simulator training or who had performed more than 2 colonoscopies in a clinical setting were excluded from this example.

Using a MEI Scope Guide (Olympus Optical, Tokyo, Japan), the route of the colonoscope (CF-H180DL, Evis Exera II video system center CV-180, Olympus Medical Systems Ltd, Tokyo, Japan) was recorded through a simulated colon in a standardized training model (Kyoto Kagaku Colonoscope Training Model).

The testing was done in a realistic setup, with a real colonoscope, real magnetic endoscopic imaging (MEI) and a realistic standardized model of the human colon.

MEI is able to determine the position of the colonoscope because the colonoscope comprises integrated coils set at regular distances, generating a pulsed low-voltage magnetic field; a magnetic sensor external to the patient/simulator detects these impulses. The signals are then modified in the detector and translated into a real-time, 3-dimensional view of the colonoscope. The image is displayed on a monitor in an anteroposterior view, lateral view, or both, seen as a split-screen view. The MEI was visible to the participants.

The Kyoto Kagaku Colonoscope Training Model is a physical model simulator mimicking the human colon, placed in a real-size plastic torso, fixed by Velcro and rubber rings. It can be configured into 6 standard cases, each case with a different loop formation and difficulty.

Both groups were introduced to the simulator, and trainees were introduced to the colonoscope by an experienced endoscopy instructor. The participants performed colonoscopy in an easy case with no loops (case 1) and a more difficult case with a loop formation of the sigmoid colon (case 2). The simulator and MEI were positioned equally in all sessions.

The colonoscope guide was calibrated and was lubricated before each colonoscopy. Participants had a maximum of 15 minutes to reach the "cecum," which was marked by an indentation made with a clothes pin.

Only the MEI data from insertion of the colonoscope to the "cecum" was recorded; not the withdrawal.

The magnetic endoscopic images were recorded directly from the Scope Guide through a medical recording device (Medicap USB200, Medicapture Inc, Plymouth Meeting, Pennsylvania, USA) at 25 Hz and saved on a separate USB storage disk for each test participant. The Scope Guide tracked at 10 Hz, and because of this, the frequency of the recorded images were decimated to 10 Hz in VirtualDub (VDubMPEG2_1.6.19, Virtualdub.org).

Based on the video recordings of the magnetic endoscopic images, a MatLab 2012a (Math Works, Inc, Natick, Massachusetts,USA) was used to identify the tip of the colonoscope for each picture. The position of the tip was entered into a grid consisting of multiple squares of 10 x 10 pixels.

The algorithm used in this example for scoring the insertion was in analogy with the flow diagram set out in fig. 4.

Accordingly, the score was reduced if the tip returned to a square already visited, signifying no progression. The score was increased with the fewer squares visited before the cecum was reached.

This ensured that an even progression would result in a high score, whereas advancing in an uneven, insecure fashion resulted in a lower score.

In addition to the CPTS, the method also includes the opportunity to build a graphical map of the progression of the procedure as a plot of the position of the colonoscope in the virtual grid.

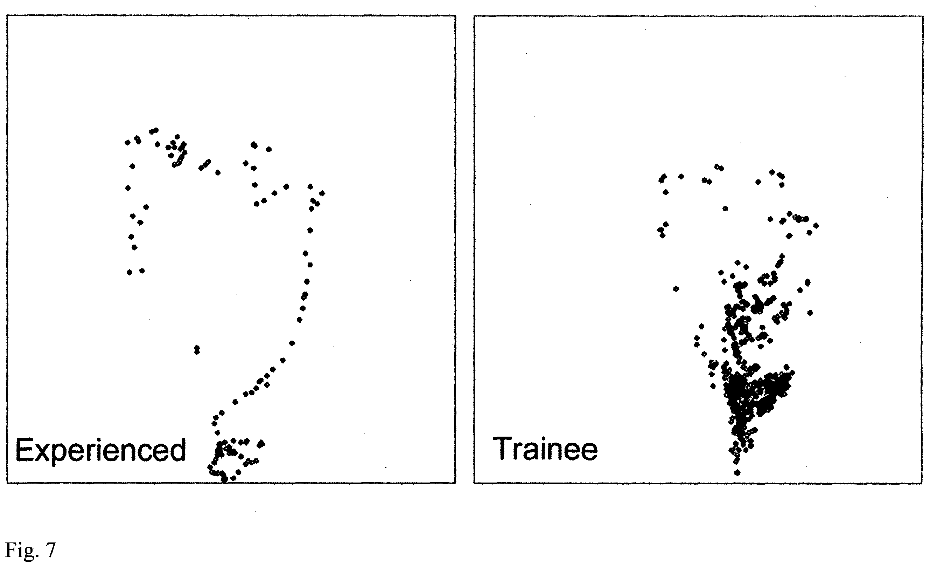

This is illustrated in fig. 7, where the position of the colonoscope was marked 5 times each second. It is clearly shown that a steady, even progression (experienced; left hand side) results in a map with equally spaced dots, almost as a "drawing" of the colon model, whereas a trainee's insecure uneven progression (right hand side) results result in a dense pattern of dots in problem areas. This colonoscopy progression map (CoP-map) may be printed and used to evaluate the training procedure, along with the CPTS.

Statistical analyses were performed with IBM SPSS Statistics (PASW, version 18.0; SPSS Inc, Chicago, Illinois, USA). For nonparametric testing of independent data, the Mann-Whitney U test was used. Significance level was set at P < 0.05. The contrasting groups method was used to determine the passing score. This was possible because the 2 groups were known to be different by external criteria (experience, number of colonoscopies). The passing standard was determined by graphing the score distributions of the 2 groups and set at the intersection of the 2 resulting curves, as the false positive (trainees passing) and false negative (experienced failing) were considered to be of equal weight.

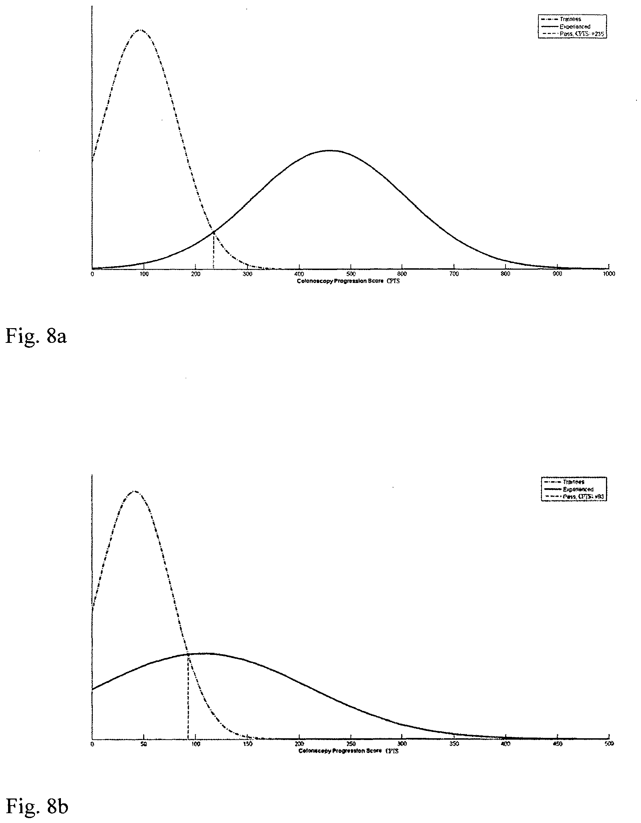

This situation is illustrated in Fig. 8a and Fig. 8b showing the distribution of scores of the trainees (broken curve) and of the experienced endoscopy consultants (solid curve), in respect of the easy case (case 1) in Fig. 8a and in respect of the difficult case (case 2) in Fig. 8b.

### Results

Twenty-one physicians participated in the study; there were no incomplete data sets or missing data. In the easier case (case 1), the difference in performance was convincing. The trainees had a mean CPTS of 93.1 (SD 73.4), whereas the experienced participants scored a mean 459.7 (SD 147.5), and the difference was found to be highly significant (P < 0.001).

In the more difficult case (case 2), the difference in performance was still significant, but some of the experienced participants did not score as high as we had predicted. The trainees achieved a mean CPTS of 41.1 (SD 35.5) and the experienced achieved a mean CPTS of 106.9 (SD 102.5), a difference that was also significant (P < 0.01). To determine the passing score, the score distributions by using the contrasting groups method and set the passing score at the intersection of the 2 resulting curves, were graphed.

This resulted in a passing score of 234.8 in case 1 (see Fig. 8a) and 93.0 in case 2 (see Fig. 8b).

In case 1, all experienced endoscopists and only 1 trainee passed. In case 2, 1 novice and only 3 experienced endoscopists passed).

This example illustrates that the apparatus according to the present invention can be used as an automated assessment tool for assessment of the quality of a colonoscopy. This tool was able to distinguish between trainee and experienced endoscopists in an easy and difficult case scenario on a physical colonoscopy simulator; it is a dynamic, fully objective instrument and offers the possibility of live feedback. Furthermore, the inventive apparatus gives a valuable assessment tool that does not require full-time procedure supervision by an experienced supervisor.

### List of reference numerals

- 2: Elongate flexible endoscopic arm
- 4: Abdominal part of a human body
- 6: Rectum
- 8: Large intestine
- 10: Stomach
- 11: Individual
- 12: Small intestine
- 14: Sensor system
- 16: Quality assessment system
- 18: Proximate end of elongate flexible endoscopic arm
- 19: Connector to control unit include in text
- 20: Distal end of elongate flexible endoscopic arm
- 22: Shape controlling input means of endoscopic arm
- 24: Means for adjusting the spatial shape of endoscopic arm
- 26: Position indicating means
- 28: Image capturing means
- 30: Image of an intestine
- 32: Control unit
- 33: 3D representation of the position of position indicating means
- 34: Monitor signal
- 36,38,50: Monitor
- 40: Selected point on elongate flexible endoscopic arm
- 42,52: Data storage
- 44: Score
- 46: Communication means for communicating one or more scores to a user
- 48: Illuminating means of endoscopic arm
- 54: Input means for inputting identity of user
- 100: Apparatus

## Claims

1. An apparatus (100) for monitoring the quality of an endoscopy, in particular a colonoscopy, performed on an individual (11), said apparatus comprises:
- an elongate flexible endoscopic arm (2) adapted to be inserted into the large intestine (4) of an individual; and
- a sensor system (14) adapted to be arranged outside the body of an individual; and
- a quality assessment system (16) for evaluating the quality of a specific endoscopy procedure performed on an individual;
wherein said endoscopic arm is being adapted to be inserted into the large intestine (8) of an individual via that individual's rectum (6), said endoscopic arm having a proximate end (18) and a distal end (20);
wherein said endoscopic arm at a position near the proximal end comprises shape controlling input means (22) for controlling the spatial shape of said arm; and
wherein said endoscopic arm along its extension comprises means (24) for adjusting the spatial shape of said arm in response to an activation of said shape controlling input means (22);
wherein said endoscopic arm along its extension comprises one or more position indicating means (26), each position indicating means being adapted to provide information as to the position of said specific position indicating means; and
wherein said endoscopic arm near its distal end (20) comprises one or more image capturing means (28) for capturing images (30) of the interior of an intestine;
wherein said sensor system (14) being adapted for sensing one or more of said position indicating means (26) of said endoscopic arm (2); said sensor system being adapted to generate an electrical signal as a representation of the position of one or more of said position indicating means (26), when said one or more position indicating means are within a detection range of said sensor system (14);
wherein said sensor system (14) comprises a control unit (32) comprising a microprocessor which is adapted to process the electrical signal originating from said sensor system (14), said control unit being adapted to translate this electrical signal into a 3D representation (33) of the position of one or more of said position indicating means (26) in the form of a monitor signal (34);
wherein said apparatus comprises a monitor (36), said monitor being adapted to display graphically said 3D representation (33) of the position of one or more of said position indicating means (26);
wherein said apparatus comprises a monitor (38), said monitor being adapted to display graphically the imaged (30) captured by the one or more image capturing means (28) of said endoscopic arm (2);
wherein said quality assessment system (16) comprises means for determining, at predefined time intervals, the position in the three dimensional space, of one or more selected points (40); said one or more selected points (40) being points fixed on the elongate flexible endoscopic arm; at least one of said one or more selected points (40) being a point located at the distal end of the elongate flexible arm;
wherein said quality assessment system (16) comprises means for determining, in respect of one or more of said selected points (40), one or more parameters relating to the progression of one or more of said selected points; said parameters being selected from the group comprising: speed of progression of a specific selected point, direction of the progression of a specific selected point, time span of non-movement of a specific selected point;
wherein said quality assessment system (16) comprises a data storage (42) for storing data associated with parameters of one or more examples of an ideal progression of an endoscopic insertion;
wherein said quality assessment system (16) comprises means for calculating one or more scores (44) by comparing one or more parameters of an actually performed endoscopy insertion with the same one or more parameters associated with one or more of said ideal progressions of an endoscopic insertion, wherein said one or more scores representing the degree of deviation from one or more selected examples of an ideal progression of an endoscopic insertion; thus allowing a quality assessment of the progression of the flexible endoscopic arm, at least as represented by the progression of a distal point of said arm, such as the tip of said arm;
wherein said quality assessment system (16) comprises communication means (46) which is adapted to communicate said one or more scores (44) to the user.

2. An apparatus according to claim 1, wherein one of said one or more selected points (40) being a point located at the distal end (20) of the elongate flexible endoscopic arm (2).

3. An apparatus according to claim 1 or 2, wherein said one or more position indicating means (26) of said endoscopic arm (2) comprises permanent magnets or electromagnets.

4. An apparatus according to any of the claims 1 - 3, wherein the number of position indicating means (26) arranged along the extension of the endoscopic arm (2) is 1-50.

5. An apparatus according to any of the claims 1 - 4, wherein said one or more image capturing means (28) of the endoscopic arm (2) for capturing images (30) of the interior of an intestine being in the form of a video camera or a lens comprising means for transmitting images to said control unit.

6. An apparatus according to any of the claims 1 - 5, wherein said elongate flexible endoscopic arm (2) comprises one or more illuminating means (48) for illuminating the interior of an intestine, said one or more illuminating means preferably being arranged at the distal part (20) of said elongate flexible endoscopic arm (2).

7. An apparatus according to any of the claims 1 - 6, wherein said monitor (36) being adapted to display graphically said 3D representation of the position of one or more of said position indicating means is the same monitor or is a different monitor as that monitor (38) being adapted to display graphically the images (30) captured by the one or more image capturing means of said endoscopic arm.

8. An apparatus according to any of the claims 1 - 7, wherein said data associated with parameters of one or more examples of an ideal progression of an endoscopic insertion are based on a single human individual (11); or wherein said data associated with parameters of one or more examples of an ideal progression of an endoscopic insertion are based on an average of a number of different human individuals (11).

9. An apparatus according to any of the claims 1 - 8, wherein said communication means (46) of said quality assessment system (16), which is adapted to communicate said one or more scores to the user, is configured to communicate said one or more scores graphically, such as by displaying colour(s) and/or by displaying numbers, preferably on a monitor (50).

10. An apparatus according to any of the claims 1 - 9, wherein said apparatus is configured so as to calculate the position of one or more of said selected points (40) in the three dimensional space directly from the information provided by the said electric signal originating from said sensor system (14); or indirectly from said monitor signal (34) provided by said control unit (32).

11. An apparatus according to any of the claims 1 - 10, wherein said data associated with parameters of one or more examples of an ideal progression of an endoscopic insertion and being stored on said data storage (42) is defined as: shortest possible time to the coecum, with no clinical obvious complain from the individual or with complain from the individual below a predefined level.

12. An apparatus according to any of the claims 1 - 11, wherein said apparatus comprises a data storage (52) for recording, as a function of time, the position of one or more of said position indicating means.

13. An apparatus according to any of the claims 1 - 12, wherein said apparatus comprises input means (54) for inputting the identity of the specific medical staff person, performing a specific endoscopy; and wherein said apparatus optionally furthermore comprises means for correlating and displaying, in respect of said specific medical staff person, an average score obtained by said identity of that specific medical staff, thus allowing obtaining a accumulated quality assessment of one or more medical staff persons.

14. Use of an apparatus (100) according to any of the claims 1 - 13 for training a medical staff person in performing an endoscopy, especially a colonoscopy, on an individual (11); with the proviso that said individual being a model of a human being or part of a model of a human being or model of a part of a human being.

15. A method for training a medical staff person in performing an endoscopy, especially colonoscopy, said method comprising:
i) providing an apparatus (100) according to any of the claims 1 -13;
ii) inserting the distal end (20) of the elongate flexible endoscopic arm (2) into the rectum (6) of an individual (11);
iii) progressively inserting the elongate flexible endoscopic arm (2) into that individual's large intestine (8) while following the internal topography of the large intestine by means of adjustment of the shape controlling input means (22) of the endoscopic arm (2), and with reference to the 3D representation of the position of one or more of said position indicating means (26) on said monitor (36);
iv) during step iii) repeatedly receiving information from said quality assessment system (16) relating to the degree of deviation from progression of an endoscopic insertion, said information being provided by said score/scores (44);
v) using the score/scores (44) obtained in step iv) as a feed-back for correcting the progression of the progression of the insertion of the endoscopic arm, and subsequently, if necessary, correcting the progression of the insertion of the endoscopic arm (2);
vi) retracting said endoscopic arm (2) from the individual's (11) body (4);
with the proviso that said individual (11) being a model of a human being or part of a model of a human being or model of a part of a human being..

## Patentansprüche

1. Vorrichtung (100) zum Überwachen der Qualität einer Endoskopie, insbesondere einer Koloskopie, die an einem Individuum (11) vorgenommen wird, wobei die Vorrichtung umfasst:
- einen länglichen flexiblen Endoskop-Arm (2), der dazu ausgebildet ist, in den Dickdarm (4) eines Individuums eingeführt zu werden; und
- ein Sensorsystem (14), das dazu ausgebildet ist, außerhalb des Körpers eines Individuums angeordnet zu werden; und
- ein Qualitätsbeurteilungssystem (16) zum Beurteilen der Qualität eines spezifischen Endoskopie-Eingriffs, der an einem Individuum vorgenommen wird;
wobei der Endoskop-Arm dazu ausgebildet ist, über das Rektum (6) eines Individuums in den Dickdarm (8) dieses Individuums eingeführt zu werden, wobei der Endoskop-Arm ein proximales Ende (18) und ein distales Ende (20) besitzt;
wobei der Endoskop-Arm an einer Position nahe des proximalen Endes Formsteuerungs-Eingabemittel (22) zum Steuern der räumlichen Form des Arms umfasst; und
wobei der Endoskop-Arm entlang seiner Erstreckung Mittel (24) zum Verstellen der räumlichen Form des Arms in Antwort auf eine Aktivierung der Formsteuerungs-Eingabemittel (22) umfasst;
wobei der Endoskop-Arm entlang seiner Erstreckung ein oder mehrere Positionsangabemittel (26) umfasst, wobei jedes Positionsangabemittel dazu ausgebildet ist, Informationen bezüglich der Position des spezifischen Positionsangabemittels bereitzustellen; und
wobei der Endoskop-Arm nahe seinem distalen Ende (20) ein oder mehrere Bilderfassungsmittel (28) zum Erfassen von Bildern (30) des Inneren eines Darms umfasst;
wobei das Sensorsystem (14) dafür ausgebildet ist, eines oder mehrere der Positionsangabemittel (26) des Endoskop-Arms (2) zu erkennen; wobei das Sensorsystem dazu ausgebildet ist, ein elektrisches Signal als eine Darstellung der Position von eines oder mehrerer der Positionsangabemittel (26) zu erzeugen, wenn sich das eine oder mehrere Positionsangabemittel innerhalb eines Detektionsbereichs des Sensorsystems (14) befinden;
wobei das Sensorsystem (14) eine Steuereinheit (32) umfasst, umfassend einen Mikroprozessor, der dazu ausgebildet ist, das aus dem Sensorsystem (14) stammende elektrische Signal zu verarbeiten, wobei die Steuereinheit dazu ausgebildet ist, dieses elektrische Signal in eine 3D-Darstellung (33) der Position eines oder mehrerer der Positionsangabemittel (26) in der Form eines Monitorsignals (34) zu übersetzen;
wobei die Vorrichtung einen Monitor (36) umfasst, wobei der Monitor dazu ausgebildet ist, die 3D-Darstellung (33) der Position eines oder mehrerer der Positionsangabemittel (26) grafisch anzuzeigen;
wobei die Vorrichtung einen Monitor (38) umfasst, wobei der Monitor dazu ausgebildet ist, die von dem einen oder mehreren Bilderfassungsmitteln (28) des Endoskop-Arms (2) erfassten Bilder (30) grafisch anzuzeigen;
wobei das Qualitätsbeurteilungssystem (16) Mittel zum Bestimmen, in vordefinierten Zeitintervallen, der Position von einem oder mehreren ausgewählten Punkten (40) im dreidimensionalen Raum umfasst; wobei der eine oder mehrere ausgewählte Punkte (40) Punkte sind, die an dem länglichen flexiblen Endoskop-Arm befestigt sind; wobei mindestens einer aus dem einen oder mehreren ausgewählten Punkten (40) ein Punkt ist, der am distalen Ende des länglichen flexiblen Arms liegt;
wobei das Qualitätsbeurteilungssystem (16) Mittel zum Bestimmen, in Bezug auf einen oder mehrere der ausgewählten Punkte (40), von einem oder mehreren Parametern in Verbindung mit der Progression von einem oder mehreren der ausgewählten Punkte umfasst; wobei die Parameter ausgewählt sind aus der Gruppe, umfassend: Progressionsgeschwindigkeit eines spezifischen ausgewählten Punkts, Richtung der Progression eines spezifischen ausgewählten Punkts, Zeitspanne der Nichtbewegung eines spezifischen ausgewählten Punkts;
wobei das Qualitätsbeurteilungssystem (16) einen Datenspeicher (42) zum Speichern von Daten umfasst, die zu Parametern von einem oder mehreren Beispielen einer idealen Progression einer Endoskop-Einführung gehören;
wobei das Qualitätsbeurteilungssystem (16) Mittel zum Berechnen von einem oder mehreren Ergebnissen (44) durch Vergleichen von einem oder mehreren Parametern einer tatsächlich vorgenommenen Endoskopie-Einführung mit dem gleichen einen oder mehreren Parametern umfasst, die zu einer oder mehreren der idealen Progressionen einer Endoskop-Einführung gehören, wobei das eine oder mehrere Ergebnisse den Abweichungsgrad von einem oder mehreren ausgewählten Beispielen einer idealen Progression einer Endoskop-Einführung darstellen; wodurch eine Qualitätsbeurteilung der Progression des flexiblen Endoskop-Arms, mindestens wie durch die Progression eines distalen Punkts des Arms, wie etwa der Spitze des Arms dargestellt, ermöglicht wird;
wobei das Qualitätsbeurteilungssystem (16) Kommunikationsmittel (46) umfasst, das dazu ausgebildet ist, das eine oder mehrere Ergebnisse (44) an den Benutzer zu kommunizieren.

2. Vorrichtung nach Anspruch 1, wobei einer aus dem einen oder mehreren ausgewählten Punkten (40) ein Punkt ist, der am distalen Ende (20) des länglichen flexiblen Endoskop-Arms (2) liegt.

3. Vorrichtung nach Anspruch 1 oder 2, wobei das eine oder mehrere Positionsangabemittel (26) des Endoskop-Arms (2) Dauermagneten oder Elektromagneten umfassen.

4. Vorrichtung nach einem der Ansprüche 1-3, wobei die Anzahl von Positionsangabemitteln (26), die entlang der Erstreckung des Endoskop-Arms (2) angeordnet sind, 1-50 beträgt.

5. Vorrichtung nach einem der Ansprüche 1-4, wobei das eine oder mehrere Bilderfassungsmittel (28) des Endoskop-Arms (2) zum Erfassen von Bildern (30) des Inneren eines Darms in der Form einer Videokamera oder einer Linse vorliegen, umfassend Mittel zum Übertragen von Bildern an die Steuereinheit.

6. Vorrichtung nach einem der Ansprüche 1-5, wobei der längliche flexible Endoskop-Arm (2) ein oder mehrere Beleuchtungsmittel (48) zum Beleuchten des Inneren eines Darms umfasst, wobei das eine oder mehrere Beleuchtungsmittel bevorzugt am distalen Teil (20) des länglichen flexiblen Endoskop-Arms (2) angeordnet sind.

7. Vorrichtung nach einem der Ansprüche 1-6, wobei der Monitor (36), der dazu ausgebildet ist, die 3D-Darstellung der Position eines oder mehrerer der Positionsangabemittel grafisch anzuzeigen, der gleiche Monitor ist oder ein verschiedener Monitor ist als derjenige Monitor (38), der dazu ausgebildet ist, die von dem einen oder mehreren Bilderfassungsmitteln des Endoskop-Arms erfassten Bilder (30) grafisch anzuzeigen.

8. Vorrichtung nach einem der Ansprüche 1-7, wobei die Daten, die zu Parametern von einem oder mehreren Beispielen einer idealen Progression einer Endoskop-Einführung gehören, auf einem einzelnen menschlichen Individuum (11) basieren; oder wobei die Daten, die zu Parametern von einem oder mehreren Beispielen einer idealen Progression einer Endoskop-Einführung gehören, auf einem Durchschnitt einer Anzahl von verschiedenen menschlichen Individuen (11) basieren.

9. Vorrichtung nach einem der Ansprüche 1-8, wobei das Kommunikationsmittel (46) des Qualitätsbeurteilungssystems (16), das dazu ausgebildet ist, das eine oder mehrere Ergebnisse an den Benutzer zu kommunizieren, dazu eingerichtet ist, das eine oder mehrere Ergebnisse grafisch, wie etwa durch Anzeigen von Farbe(n) und/oder durch Anzeigen von Zahlen, bevorzugt auf einem Monitor (50), zu kommunizieren.

10. Vorrichtung nach einem der Ansprüche 1-9, wobei die Vorrichtung so eingerichtet ist, dass sie die Position von einem oder mehreren der ausgewählten Punkte (40) im dreidimensionalen Raum direkt aus den Informationen, die von dem aus dem Sensorsystem (14) stammenden elektrischen Signal bereitgestellt werden, berechnet; oder indirekt aus dem von der Steuereinheit (32) bereitgestellten Monitorsignal (34).

11. Vorrichtung nach einem der Ansprüche 1-10, wobei die Daten, die zu Parametern von einem oder mehreren Beispielen einer idealen Progression einer Endoskop-Einführung gehören und auf dem Datenspeicher (42) gespeichert sind, definiert sind als: kürzest mögliche Zeit bis zum Blinddarm, ohne klinisch offensichtliche Beschwerden des Individuums oder mit Beschwerden des Individuums unterhalb eines vordefinierten Niveaus.

12. Vorrichtung nach einem der Ansprüche 1-11, wobei die Vorrichtung einen Datenspeicher (52) zum Aufzeichnen, in Abhängigkeit von der Zeit, der Position von einem oder mehreren der Positionsangabemittel umfasst.

13. Vorrichtung nach einem der Ansprüche 1-12, wobei die Vorrichtung Eingabemittel (54) zum Eingeben der Identität des spezifischen Mitarbeiters des medizinischen Personals, der eine spezifische Endoskopie vornimmt, umfasst; und wobei die Vorrichtung gegebenenfalls weiter Mittel zum Korrelieren und Anzeigen, in Bezug auf den spezifischen Mitarbeiter des medizinischen Personals, eines Durchschnittsergebnisses umfasst, der von der Identität dieses spezifischen medizinischen Personals erzielt wurde, wodurch das Erzielen einer kumulierten Qualitätsbeurteilung von einem oder mehreren Mitarbeitern des medizinischen Personals ermöglicht wird.

14. Verwendung einer Vorrichtung (100) nach einem der Ansprüche 1-13 zum Schulen eines Mitarbeiters des medizinischen Personals im Vornehmen einer Endoskopie, insbesondere einer Koloskopie, an einem Individuum (11); unter der Voraussetzung, dass das Individuum ein Modell eines Menschen oder Teil eines Modells eines Menschen oder Modell eines Teils eines Menschen ist.

15. Verfahren zum Schulen eines Mitarbeiters des medizinischen Personals im Vornehmen einer Endoskopie, insbesondere Koloskopie, wobei das Verfahren umfasst:
i) Bereitstellen einer Vorrichtung (100) nach einem der Ansprüche 1-13;
ii) Einführen des distalen Endes (20) des länglichen flexiblen Endoskop-Arms (2) in das Rektum (6) eines Individuums (11);
iii) progressives Einführen des länglichen flexiblen Endoskop-Arms (2) in den Dickdarm (8) dieses Individuums unter Verfolgung der internen Topographie des Dickdarms mittels Verstellen der Formsteuerungs-Eingabemittel (22) des Endoskop-Arms (2), und unter Bezugnahme auf die 3D-Darstellung der Position eines oder mehrerer der Positionsangabemittel (26) auf dem Monitor (36);
iv) wiederholtes Empfangen, während Schritt iii), von Informationen von dem Qualitätsbeurteilungssystem (16) in Verbindung mit dem Abweichungsgrad von Progression einer Endoskop-Einführung, wobei die Informationen durch das Ergebnis/die Ergebnisse (44) bereitgestellt werden;
v) Verwenden des/der in Schritt iv) erzielten Ergebnisses/Ergebnisse (44) als ein Feedback, zum Korrigieren der Progression, der Progression des Einführens des Endoskop-Arms, und anschließend, falls erforderlich, Korrigieren der Progression des Einführens des Endoskop-Arms (2);
vi) Herausziehen des Endoskop-Arms (2) aus dem Körper (4) des Individuums (11);
unter der Voraussetzung, dass das Individuum (11) ein Modell eines Menschen oder Teil eines Modells eines Menschen oder Modell eines Teils eines Menschen ist.

## Revendications

1. Appareil (100) de surveillance de la qualité d'une endoscopie, en particulier d'une colonoscopie, réalisée sur un individu (11), ledit appareil comprend :
- un bras endoscopique flexible allongé (2) adapté pour être inséré dans le gros intestin (4) d'un individu ; et
- un système de capteur (14) adapté pour être agencé à l'extérieur du corps d'un individu ; et
- un système d'estimation de qualité (16) pour évaluer la qualité d'une procédure d'endoscopie spécifique réalisée sur un individu ;
dans lequel ledit bras endoscopique est adapté pour être inséré dans le gros intestin (8) d'un individu via le rectum (6) de cet individu, ledit bras endoscopique ayant une extrémité proximale (18) et une extrémité distale (20) ;
dans lequel ledit bras endoscopique à une position près de l'extrémité proximale comprend des moyens d'entrée de contrôle de forme (22) pour contrôler la forme spatiale dudit bras ; et
dans lequel ledit bras endoscopique le long de son extension comprend des moyens (24) pour ajuster la forme spatiale dudit bras en réponse à une activation desdits moyens d'entrée de contrôle de forme (22) ;
dans lequel ledit bras endoscopique le long de son extension comprend un ou plusieurs moyens d'indication de position (26), chaque moyen d'indication de position étant adapté pour fournir des informations quant à la position desdits moyens d'indication de position spécifiques ; et
dans lequel ledit bras endoscopique près de son extrémité distale (20) comprend un ou plusieurs moyens de capture d'image (28) pour capturer des images (30) de l'intérieur d'un intestin ;
dans lequel ledit système de capteur (14) étant adapté pour capter un ou plusieurs desdits moyens d'indication de position (26) dudit bras endoscopique (2) ; ledit système de capteur étant adapté pour générer un signal électrique en tant que représentation de la position d'un ou de plusieurs desdits moyens d'indication de position (26), lorsque lesdits un ou plusieurs moyens d'indication de position entrent dans une portée de détection dudit système de capteur (14) ;
dans lequel ledit système de capteur (14) comprend une unité de commande (32) comprenant un microprocesseur qui est adapté pour traiter le signal électrique ayant pour origine ledit système de capteur (14), ladite unité de commande étant adaptée pour traduire ce signal électrique en une représentation 3D (33) de la position d'un ou de plusieurs desdits moyens d'indication de position (26) sous la forme d'un signal de moniteur (34) ;
dans lequel ledit appareil comprend un moniteur (36), ledit moniteur étant adapté pour afficher graphiquement ladite représentation 3D (33) de la position d'un ou de plusieurs desdits moyens d'indication de position (26) ;
dans lequel ledit appareil comprend un moniteur (38), ledit moniteur étant adapté pour afficher graphiquement l'image (30) capturée par les un ou plusieurs moyens de capture d'image (28) dudit bras endoscopique (2) ;
dans lequel ledit système d'estimation de qualité (16) comprend un moyen pour déterminer, à des intervalles de temps prédéfinis, la position dans l'espace tridimensionnel, d'un ou de plusieurs points sélectionnés (40) ; lesdits un ou plusieurs points sélectionnés (40) étant des points fixes sur le bras endoscopique flexible allongé ; au moins l'un desdits un ou plusieurs points sélectionnés (40) étant un point situé à l'extrémité distale du bras flexible allongé ;
dans lequel ledit système d'estimation de qualité (16) comprend un moyen pour déterminer, quant aux un ou plusieurs dits points sélectionnés (40), un ou plusieurs paramètres relatifs à la progression d'un ou de plusieurs desdits points sélectionnés ; lesdits paramètres étant sélectionnés dans le groupe comprenant : la vitesse de progression d'un point sélectionné spécifique, la direction de la progression d'un point sélectionné spécifique, le laps de temps d'un non-mouvement d'un point sélectionné spécifique ;
dans lequel ledit système d'estimation de qualité (16) comprend une mémoire de données (42) pour stocker des données associées à des paramètres d'un ou de plusieurs exemples d'une progression idéale d'une insertion endoscopique ;
dans lequel ledit système d'estimation de qualité (16) comprend des moyens pour calculer un ou plusieurs scores (44) en comparant un ou plusieurs paramètres d'une insertion d'endoscopie réalisée effectivement avec les mêmes un ou plusieurs paramètres associés à une ou plusieurs desdites progressions idéales d'une insertion endoscopique, dans lequel lesdits un ou plusieurs scores représentant le degré d'écart par rapport à un ou plusieurs exemples sélectionnés d'une progression idéale d'une insertion endoscopique ; permettant ainsi une estimation de qualité de la progression du bras endoscopique flexible, au moins tel que représentée par la progression d'un point distal dudit bras, tel que l'embout dudit bras ;
dans lequel ledit système d'estimation de qualité (16) comprend un moyen de communication (46) qui est adapté pour communiquer lesdits un ou plusieurs scores (44) à l'utilisateur.

2. Appareil selon la revendication 1, dans lequel lesdits un ou plusieurs points sélectionnés (40) étant un point situé à l'extrémité distale (20) du bras endoscopique flexible allongé (2).

3. Appareil selon la revendication 1 ou 2, dans lequel lesdits un ou plusieurs moyens d'indication de position (26) dudit bras endoscopique (2) comprennent des aimants permanents ou des électroaimants.

4. Appareil selon l'une quelconque des revendications 1 à 3, dans lequel le nombre de moyens d'indication de position (26) agencés le long de l'extension du bras endoscopique (2) est de 1 à 50.

5. Appareil selon l'une quelconque des revendications 1 à 4, dans lequel lesdits un ou plusieurs moyens de capture d'image (28) du bras endoscopique (2) pour capturer des images (30) de l'intérieur d'un intestin se présentant sous la forme d'une caméra vidéo ou d'une lentille comprenant des moyens de transmission d'images à ladite unité de commande.

6. Appareil selon l'une quelconque des revendications 1 à 5, dans lequel ledit bras endoscopique flexible allongé (2) comprend un ou plusieurs moyens d'illumination (48) pour illuminer l'intérieur d'un intestin, lesdits un ou plusieurs moyens d'illumination étant de préférence agencés au niveau de la partie distale (20) dudit bras endoscopique flexible allongé (2).

7. Appareil selon l'une quelconque des revendications 1 à 6, dans lequel ledit moniteur (36) qui est adapté pour afficher graphiquement ladite représentation 3D de la position d'un ou de plusieurs desdits moyens d'indication de position est le même moniteur ou est un moniteur différent du moniteur (38) qui est adapté pour afficher graphiquement les images (30) capturées par les un ou plusieurs moyens de capture d'image dudit bras endoscopique.

8. Appareil selon l'une quelconque des revendications 1 à 7, dans lequel lesdites données associées aux paramètres d'un ou de plusieurs exemples d'une progression idéale d'une insertion endoscopique sont basées sur un individu humain unique (11) ; ou dans lequel lesdites données associées à des paramètres d'un ou de plusieurs exemples d'une progression idéale d'une insertion endoscopique sont basées sur une moyenne d'un certain nombre d'individus humains différents (11).

9. Appareil selon l'une quelconque des revendications 1 à 8, dans lequel ledit moyen de communication (46) dudit système d'estimation de qualité (16), qui est adapté pour communiquer lesdits un ou plusieurs scores à l'utilisateur, est configuré pour communiquer lesdits un ou plusieurs scores graphiquement, tel qu'en affichant une ou des couleurs et/ou en affichant des nombres, de préférence sur un moniteur (50).

10. Appareil selon l'une quelconque des revendications 1 à 9, dans lequel ledit appareil est configuré de façon à calculer la position d'un ou de plusieurs desdits points sélectionnés (40) dans l'espace tridimensionnel directement à partir des informations fournies par ledit signal électrique ayant pour origine ledit système de capteur (14) ; ou indirectement à partir dudit signal de moniteur (34) fourni par ladite unité de commande (32).

11. Appareil selon l'une quelconque des revendications 1 à 10, dans lequel lesdites données associées à des paramètres d'un ou de plusieurs exemples d'une progression idéale d'une insertion endoscopique et qui sont stockées sur ladite mémoire de données (42) sont définies par : le plus court temps possible jusqu'au caecum, avec aucune plainte évidente clinique de l'individu ou avec une plainte de l'individu en-dessous d'un niveau prédéfini.

12. Appareil selon l'une quelconque des revendications 1 à 11, dans lequel ledit appareil comprend une mémoire de données (52) pour enregistrer, comme une fonction du temps, la position d'un ou de plusieurs desdits moyens d'indication de position.

13. Appareil selon l'une quelconque des revendications 1 à 12, dans lequel ledit appareil comprend des moyens d'entrée (54) pour entrer l'identité d'une personne spécifique du personnel médical, réalisant une endoscopie spécifique ; et dans lequel ledit appareil comprend de surcroît facultativement des moyens pour corréler et afficher, quant à ladite personne spécifique du personnel médical, un score moyen obtenu par ladite identité de ce personnel médical spécifique, permettant ainsi d'obtenir une estimation de qualité accumulée d'une ou de plusieurs personnes du personnel médical.

14. Utilisation d'un appareil (100) selon l'une quelconque des revendications 1 à 13 pour former une personne du personnel médical dans la réalisation d'une endoscopie, en particulier d'une colonoscopie, sur un individu (11) ; à condition que ledit individu soit un modèle d'un être humain ou une partie d'un modèle d'un être humain ou un modèle d'une partie d'un être humain.

15. Procédé de formation d'une personne de personnel médical dans la réalisation d'une endoscopie, en particulier d'une colonoscopie, ledit procédé comprenant :
i) la fourniture d'un appareil (100) selon l'une quelconque des revendications 1 à 13;
ii) l'insertion de l'extrémité distale (20) du bras endoscopique flexible allongé (2) dans le rectum (6) d'un individu (11) ;
iii) l'insertion progressive du bras endoscopique flexible allongé (2) dans le gros intestin (8) de cet individu tout en suivant la topographie interne du gros intestin au moyen d'un ajustement des moyens d'entrée de contrôle de forme (22) du bras endoscopique (2), et en référence à la représentation 3D de la position d'un ou de plusieurs desdits moyens d'indication de position (26) sur ledit moniteur (36) ;
iv) pendant l'étape iii), la réception répétée d'informations depuis ledit système d'estimation de qualité (16) relatives au degré d'écart par rapport à une progression d'une insertion endoscopique, lesdites informations étant fournies par le ou lesdits score(s) (44) ;
v) l'utilisation du ou des score(s) (44) obtenu(s) à l'étape iv) comme retour pour corriger la progression de la progression de l'insertion du bras endoscopique, et ultérieurement, si nécessaire, corriger la progression de l'insertion du bras endoscopique (2) ;
vi) le retrait dudit bras endoscopique (2) du corps (4) de l'individu (11) ;
à condition que ledit individu (11) soit un modèle d'un être humain ou une partie d'un modèle d'un être humain ou un modèle d'une partie d'un être humain.
